# EUROPEAN PATENT APPLICATION

(11) **EP 4 332 578 A1**
(43) Date of publication of application: **06.03.2024**
(21) Application number: 21962611.6
(22) Date of filing: 17.11.2021
(51) Int. Cl.: G01N 35/00, G01N 33/543

(54) **METHOD FOR ANALYZING LIQUID IMMUNE RESPONSE USING MAGNETIC BEADS**

(30) Priority: 26.10.2021 KR 20210144086
(71) Applicant: Boditech Med Inc., Chuncheon-si, Gangwon-do 24398 (KR)
(72) Inventor: KIM, Hyung Hoon, Chuncheon-si Gangwon-do 24414 (KR); CHO, Chu Hyun, Chuncheon-si Gangwon-do 24399 (KR); OH, Young Jin, Chuncheon-si Gangwon-do 24434 (KR); JUNG, Ji Woon, Chuncheon-si Gangwon-do 24278 (KR); KANG, Kyung Joon, Chuncheon-si Gangwon-do 24214 (KR)
(74) Representative: Louis Pöhlau Lohrentz
(86) International application number: PCT/KR2021/016852
(87) International publication number: WO 2023/074988

(57) **Abstract**

Disclosed is an automated liquid-phase immunoassay apparatus used with a cuvette having a plurality of chambers containing a reagent necessary for detection of an analyte in a biological specimen. The apparatus includes a movable cuvette module equipped with the cuvette, an optical reading module for optical assaying of a material resulting from a reaction between the specimen and the reagent, and a dispenser module which is positioned on the cuvette module and which dispenses the specimen and the reagent to the plurality of chambers of the cuvette and washes the specimen and the reagent therefrom.

## Description

### FIELD OF THE INVENTION

The present invention relates to a liquid-phase immunoassay method using magnetic beads, and more specifically to an immune response analysis method with improved reproducibility.

### BACKGROUND ART

With the advancement of medicine, biotechnology, and various related technologies, inspections for detecting various molecular indicators such as blood cells, genes, proteins, antigens, pathogens, etc. in biological specimens such as urine and blood are being widely performed. The inspection process is generally performed by sampling a specimen, reacting the sampled specimen with a predetermined reagent suitable for a target indicator, and assaying and observing the change that occurs. This allows qualitative and/or quantitative assays of the various molecular indicators contained in the specimen, and information on the diagnosis, progress states, or prognosis of diseases may be obtained based on such assays.

One of the technologies widely used in such an inspection process is an immune response technology called EIA (enzyme immunoassay) based on specific binding between antigen and antibody. Examples thereof include color-change measurement method (chromogenic or colorimetric) for measuring a chromogenic reaction using absorbance, chemiluminescence and fluorometry, depending on the type of substrates used for the detection of the analyte. Other examples thereof include sandwich-type immune response analysis which is also called an enzyme-linked immunosorbent assay method, and competitive-type immune response analysis, depending on the method of assay.

In these assays, it is preferable to remove non-specific biological reactants for detection with high sensitivity and high specificity regardless of the method that is used. That is, in order to accurately detect the material resulting from the reaction after the reaction between the reagent and the specimen in the inspection process, it is necessary to purify or separate the material resulting from the reaction. However, in many cases, the detection of reaction results requires the use of a membrane such as nitrocellulose or the use of a two-dimensional flat plate. However, the use of such membranes or plates not only limits the reaction area, but also makes it difficult to remove non-specific reactants.

A method using magnetic beads has been proposed to effectively remove non-specific biological reactants. The method using magnetic beads removes non-specific biological reactants by repeatedly dispersing and collecting magnetic beads during the assay process. However, magnetic beads are lost during the assay process or act as an obstacle to signal detection, causing important problems that reduce the reproducibility of measured values.

### DETAILD DESCRIPTION OF THE INVENTION

### TECHNICAL PROBLEM

Therefore, the present invention is to improve signal reproducibility in a liquid-phase immunoassay method that utilize magnetic beads to remove non-specific biological reactants.

### TECHNICAL SOLUTION

According to one aspect of the present invention, a method for analyzing immune response using magnetic beads may be provided. The method may comprise (a) dispersing magnetic beads in a detection chamber; and (b) collecting the magnetic beads and separating them from the detection chamber, and then performing an optical inspection on the detection chamber.

The method comprises further (c) attaching a reagent to the magnetic beads and injecting a biological sample into a reaction chamber in which the magnetic beads are stored to bind an analyte in the biological sample to the reagent in an antigen-antibody reaction. Also, the method comprises further (d) introducing a washing tip having a magnetic beam therein into the reaction chamber to capture the magnetic beads in the reaction chamber on a surface of the washing tip.

The method comprises further (e) washing a non-specific biological sample attached to the surface of the washing tip with a washing solution in a washing chamber by repeatedly moving up and down the washing tip in the washing chamber in a state where the magnetic beam is located in the washing tip.

The dispersing the magnetic beads in the detection chamber may comprise (a-1) moving the washing tip from which the non-specific biological sample is washed away to the detection chamber together with the magnetic beam; and (a-2) separating the magnetic beam from the washing tip.

The separating the magnetic beads from the detection chamber may comprise (b-1) attaching the magnetic beads dispersed in the detection chamber to the surface of the washing tip by placing the magnetic beam inside the washing tip; and (b-2) moving the washing tip to which the magnetic beads are attached from the detection chamber together with the magnetic beam.

According to another aspect of the present invention, a method for analyzing immune response using magnetic beads may be provided. The method may comprise (a) injecting a biological sample into a reaction chamber in which magnetic beads to which reagents are attached are stored, and binding the magnetic beads with an analyte in the biological sample through an antigen-antibody reaction; (b) moving a washing tip to the reaction chamber; (c) positioning a magnetic beam at a bottom of the washing tip by moving the magnetic beam down into a hollow portion of the washing tip; (d) collecting the magnetic beads in the reaction chamber by attaching the magnetic beads to the surface of the washing tip; (e) moving the washing tip to which the magnetic beads are attached to a washing chamber; (f) physically washing a non-specific biological sample attached to the surface of the washing tip in the washing chamber; (g) moving the washing tip from which the non-specific biological sample is washed away to a detection chamber; (h) dispersing the magnetic beads attached to the surface of the washing tip into the detection chamber by moving up the magnetic beam located in the hollow portion of the washing tip; (i) attaching the magnetic beads dispersed in the detection chamber to the surface of the washing tip by moving the magnetic beam down into the hollow portion of the washing tip; and (j) moving the washing tip to which the magnetic beads are attached from the detection chamber together with the magnetic beam, and then performing an optical inspection on the detection chamber.

### TECHNICAL EFFECTS

According to the present invention, it is possible to effectively collect and disperse magnetic bead immune complexes in the process of immune response analysis using an automated washing and analysis device that repeatedly collect and disperse magnetic beads.

Furthermore, according to the present invention, the coefficient of variation (CV) is reduced by minimizing the loss of the magnetic bead complexes, thereby improving reproducibility.

Furthermore, according to the present invention, the magnetic bead immune complexes can be removed after the reaction between the enzyme and the substrate has proceeded for a predetermined period, thereby reducing the bias signal (background signal) increased by the magnetic beads. This reduces the coefficient of variation due to the bias signal and thus improves reproducibility. In addition, the amount of light absorbed and scattered by the magnetic beads can be maximized, ultimately increasing the signal value emitted after the reaction.

### BRIEF DESCRIPTION OF THE DRAWINGS

FIG. 1 is a schematic diagram of a liquid-phase immunoassay apparatus according to an embodiment of the present invention.
FIG. 2A and 2B illustrate a process of sandwich immune response using magnetic beads.
FIG. 3A and 3A illustrate a process of competitive immune response using magnetic beads.
FIG. 4 shows a structure of a cuvette according to an embodiment of the present invention.
FIG. 5 is a schematic diagram of a dispensing tip according to an embodiment of the present invention.
FIG. 6 is a schematic diagram of a washing tip according to an embodiment of the present invention.
FIG. 7 illustrates a state of a cuvette equipped with a dispensing tip and a washing tip, according to an embodiment of the present invention.
FIG. 8 and 9 illustrate the liquid-phase immunoassay apparatus with the housing omitted, according to an embodiment of the present invention.
FIG. 10 is a schematic diagram of the holder in a liquid-phase immunoassay apparatus according to an embodiment of the present invention.
FIG. 11 illustrates a state in which the cuvette shown in FIG. 7 is mounted on the holder shown in FIG. 10.
FIG. 12 illustrates the lower part of the holder shown in FIG. 10.
FIG. 13 is a schematic diagram of the remover module in the liquid-phase immunoassay apparatus according to an embodiment of the present invention.
FIG. 14 is a view of the liquid-phase immunoassay apparatus shown in FIG. 8 with the rear frame omitted.
FIG. 15 and 16 are schematic diagrams of a dispenser module according to an embodiment of the present invention.
FIG. 17 is a schematic diagram of a dispenser module according to another embodiment of the present invention.
FIG. 18 is a detailed view of portion A shown in FIG. 17.
FIG. 19 is a partial cross-sectional view of a dispenser module according to another embodiment of the present invention.
FIG. 20 illustrates the positional relationship between the washing tip and the magnetic beam in the dispenser module shown in FIG. 19.
FIG. 21 illustrates the relationship between the distance from the permanent magnet located at the end of the magnetic beam and the magnetic flux density.
FIG. 22 illustrates the positional relationship between the washing tip and the magnetic beam in the reaction chamber, washing chamber, and detection chamber according to an embodiment of the present invention.
FIG. 23 is an overall flow chart of a liquid-phase immunoassay method according to an embodiment of the present invention.
FIG. 24 is a flowchart of a sample dispensing process according to an embodiment of the present invention.
FIG. 25 is a flow chart of a washing process according to an embodiment of the present invention.
FIG. 26 is a flowchart of an optical inspection process according to an embodiment of the present invention.
FIG. 27A illustrates measured values of reproducibility and bias according to a conventional optical inspection process, and FIG. 27B illustrates measured values of reproducibility and bias according to an embodiment of the present invention.

### BEST MODE FOR ACCOMPLISHING THE INVENTION

Hereinafter, preferred embodiments according to the present invention will be described with reference to the accompanying drawings. The present embodiments are illustrative and not intended to limit the invention in any way.

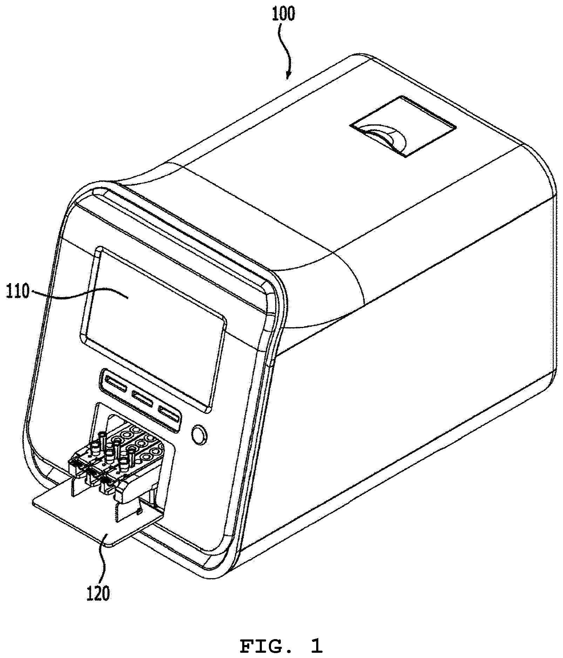

FIG. 1 is a schematic diagram of a liquid-phase immunoassay apparatus 1 according to an embodiment of the present invention. As shown, the liquid-phase immunoassay apparatus 1 has a display portion 110 and an inlet and outlet 120 in the housing 100.

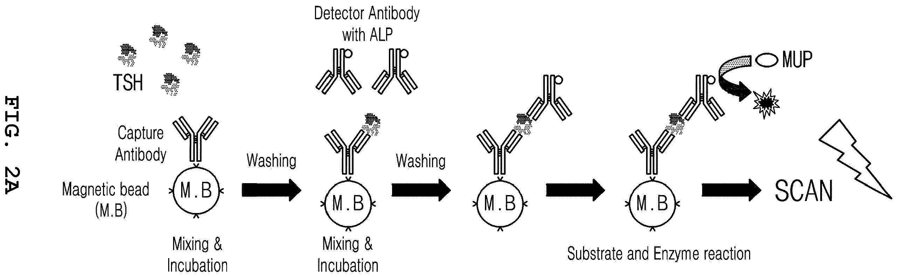

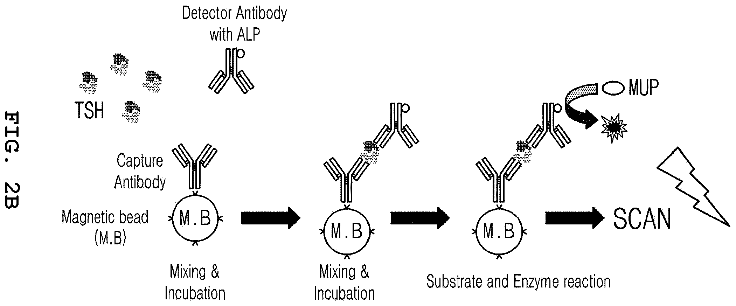

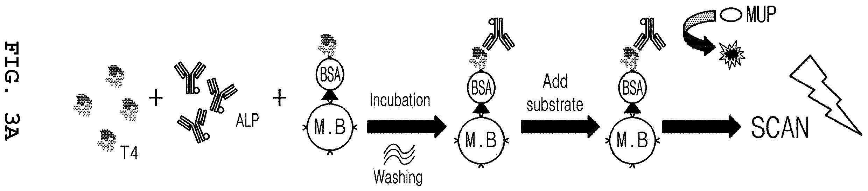

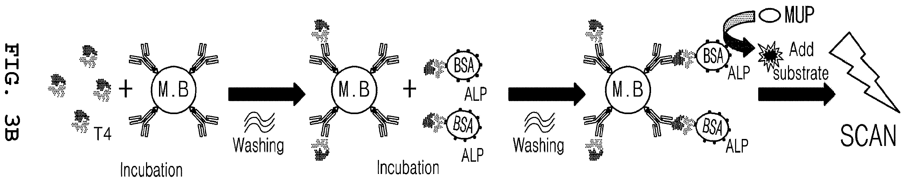

FIGS. 2A and 2B are diagrams illustrating a sandwich immunoassay using magnetic beads in an enzyme-linked immunosorbent assay (ELISA), and FIGS. 3A and 3B are diagrams illustrating a competitive immunoassay using magnetic beads.

The sandwich immunoassay is an immunoreaction in which a capture antibody and a detector antibody are bonded in a sandwich, where the detector antibody is chemically coupled to an enzyme to induce a quantitative reaction with a substrate. The capture antibody is chemically or physically bonded to magnetic beads, and the detector antibody adopts a conjugate bonded to the enzyme. The sandwich immunoassay using magnetic beads may be broadly divided into two types, and is divided into a one-step reaction (one-step assay) or a two-step reaction (two-step assay) depending on the number of washing steps. As shown in FIG. 2A, the method of reacting the sample with the capture antibody first, washing, and then reacting the detector antibody is called a two-step reaction. As shown in FIG. 2B, the method of simultaneously reacting the capture antibody and the detector antibody without distinction is called a one-step reaction.

The competition immunoassay (competition assay), which is frequently used to detect a small amount of protein molecules together with the sandwich immunoreaction, is also divided into two methods. The competition immunoassay is divided into an indirect competition reaction or a direct competition reaction depending on whether competitive proteins or antibodies are conjugated to magnetic beads, and is divided into a one-step reaction and a two-step reaction depending on the number of steps of the immunoreaction. FIG. 3A shows an indirect competition immunoassay, and FIG. 3B shows a direct competition immunoassay.

In the embodiment according to the present invention, a fluorescent signal is used to detect the material resulting from the reaction. In this case, for example, an enzyme-substrate reaction including ALP (alkaline phosphatase) and MUP (4-methylumbelliferyl phosphate) is used. ALP, which is a kind of enzyme, is a representative enzyme that causes a dephosphorylation reaction. 4-MUP is reacted with ALP to thus perform irreversible dephosphorylation due to enzymatic hydrolysis, which generates 4-MU (4-methylumbelliferone). 4-MU (4-methylumbelliferone) is excited at a wavelength of 360 nm to thus have characteristic fluorescence whereby a wavelength of 450 nm is emitted. The intensity of this fluorescent signal is detected and then used to determine the concentration of the analyte in the specimen.

In another embodiment of the present invention, color changes (colorimetric methods) are used to detect the material resulting from the reaction. The color change assay serves to detect a change in the visible color with respect to absorption of light by the material resulting from the reaction at a specific visible-ray wavelength, the absorbance is detected using the signal of the material resulting from the reaction, and the detected value is used to determine the concentration of the analyte in the specimen. For example, representative examples of enzymes and substrates may include peroxidases and substrates thereof, that is, TMB (3,3',5,5' tetramethylbenzidine), DAB (3,3',4,4' diaminobenzidine), 4CN (4-chloro-1-naphthol), ABTS (2,2'-azino-di[3-ethyl-benzthiazoline]sulfonate), and OPD (o-phenylenediamine), but are not limited thereto. For example, when TMB is used as a substrate, a blue color is generated, which may be detected using light having a wavelength of 650 nm. ABTS generates a blue-green color, which may be detected using light of 405 to 410 nm. Other examples of the enzymes and the substrates may include ALP and substrates thereof, that is, BCIP/NBT (5-bromo-4-chloro-3-indolyl-phosphate/nitroblue tetrazolium) and p-NPP (p-nitro-phenylphosphate), but are not limited thereto. These examples generate a deep yellow color, which may be detected using light having a wavelength of 405 to 410 nm.

In yet another embodiment of the present invention, chemiluminescence is used to detect the material resulting from the reaction. The chemiluminescence is the light emitted while excited electrons generated by chemical reactions return to a ground state. A light source is not required, and the chemiluminescence is measured in RLUs (relative light units) per time to determine the concentration of the analyte in the specimen. For example, examples of enzymes and substrates may include peroxidases and substrates thereof, such as luminol, polyphenols (including, for example, pyrogallol, purpurogallin, gallic acid, and umbelliferone), and acridine esters or luciferin (also referred to as bioluminescence, if used), but are not limited thereto. Other examples of the enzymes and the substrates may include ALP and AMPPD (3-(2'-spiroadamantyl)-4-methoxy-4-(3"-phosphoryloxy)-phenyl-1,2-dioxetane), but are not limited thereto.

In this assay, particularly, high-sensitivity and high-specificity detection is required, and for this, the removal of non-specific or unreacted materials is required. That is, in the inspection process, the materials resulting from the reaction need to be purified or separated in order to accurately detect the materials resulting from the reaction between the reagent and the specimen, and the apparatus according to this embodiment is an apparatus optimized for effective removal of such unreacted materials.

Specifically, in the embodiment, the apparatus according to this embodiment is an apparatus that is optimized for detection of the signal of the material resulting from the reaction obtained by removing the unreacted materials using physical washing with magnetism, separating only the material resulting from the specific reaction using a permanent magnet to thus form magnetic beads so that concentration is performed, selectively bonding a detector, to which an enzyme is attached, to the material resulting from this reaction, and finally reacting the enzyme with the substrate.

The above-described reaction used in the apparatus according to this embodiment is performed in a liquid state in a cuvette equipped in the apparatus. The apparatus according to this embodiment is optimized for performing the above-described reaction in the cuvette and for performing the reaction steps optimized in consideration of the characteristics of various parameters of the reaction performed to detect the material resulting from the reaction.

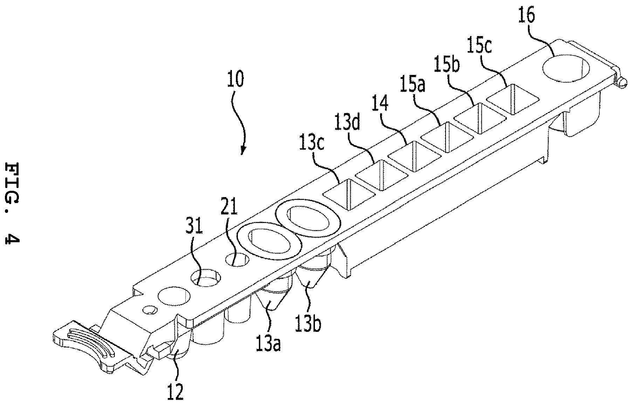

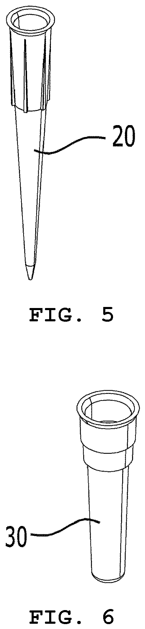

FIG. 4 is a diagram illustrating the structure of a cuvette 10 according to one embodiment of the present invention. FIG. 5 is a diagram of a dispensing tip 20, and Fig. 6 is a diagram of a washing tip 30 according to one embodiment of the present invention.

The cuvette 10 used in the automated liquid-phase immunoassay apparatus 1 according to this embodiment is used for the reaction for the detection of the analyte contained in the specimen. In the cuvette, the reaction between the specimen and the reagent is performed, and the material resulting from the reaction is generated and washed.

The cuvette 10 used in the automated liquid-phase immunoassay apparatus 1 according to this embodiment may have an elongated shape extending in forward and backward directions, as shown in FIGS. 4 and 6. Further, the cuvette 10 may include one or more insertion holes and a plurality of chambers. A chamber may also be referred to as a well.

The insertion hole is a place where the washing tip 30 and the dispensing tip 20 are inserted and wait until the inspection starts or during the inspection process. A washing tip insertion hole 21 and a dispensing tip insertion hole 31 are provided.

The chambers may comprise, in order, a specimen-filling chamber 12, chambers 13a, 13b, 13c, and 13d for a buffer solution and dilution, a reaction chamber 14, a washing chamber 15, and a detection chamber 16. The chambers may be sealed by a predetermined sealing membrane (not shown) to prevent denaturation or contamination of the reagents.

The specimen-filling chamber 12 may be provided so as to be filled with various specimens, for example, a target biological specimen to be assayed, and may be formed at the front or rear of the washing-tip-insertion hole 21 and the dispensing-tip-insertion hole 31, as described above.

The buffer solution (also referred to as a buffer) and dilution chambers 13a, 13b, 13c and 13d are filled with a magnetic (magnetic bead, MB) buffer, a detection buffer, and a specimen dilution buffer necessary for the reaction (13a, 13b, and 13c), and are provided at the rear of the specimen-filling chamber 12 or the washing-tip-insertion hole 21 and the dispensing-tip-insertion hole 31 according to the above-described sequence so that the specimen is diluted (13d).

The reaction chamber 14 is provided so as to perform the reaction between the specimen and the reagent, and is formed at the rear of the chambers for the buffer solution and dilution.

The washing chamber 15 may include a plurality of chambers in which the material resulting from the reaction is washed after the reaction in the reaction chamber. In the embodiment, three chambers 15a, 15b, and 15c are included.

In the detection chamber 16, the material resulting from the reaction, which is generated due to the reaction between the specimen and the reagent, is detected. The detection chamber 16 is provided so as to detect the presence of the analyte in the material resulting from the reaction after washing in the washing chamber 15. The detection chamber 16 may be formed at the rear of the washing chamber 15, and may be light-transmissive to enable detection of a fluorescent signal.

In the embodiment, the cuvette 10 may further include a bar code or a QR code (not shown), which is used while being associated with a chip, to be described below, inserted into the automated liquid-phase immunoassay apparatus 1 of the present invention. In the present invention, the bar code includes UPC-A, UPC-E, EAN, Code 3 of 9, Interleaved 2 of 5, Code 128, UCC/EAN-128, Codabar, PostNet, Pharmacode, or PDF-417, but is not limited thereto, or the bar code includes a 1D bar code or a 2D bar code, but is not limited thereto. The bar code or the QR code is obtained by encoding the type of the analyte depending on the type of the specimen.

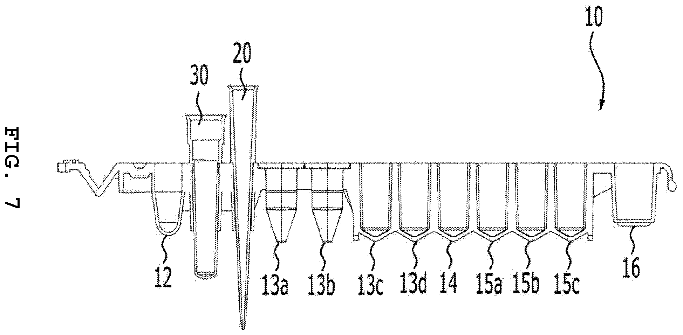

FIG. 7 illustrates a state of a cuvette 10 equipped with a dispensing tip 20 and a washing tip 30, according to an embodiment of the present invention.

The dispensing tip 20 may include a disposable micro-tip (for example, a micropipette tip having a volume of 2 to 1000 µl) which is used while being fastened to a sampling arm 556, as will be described later, in order to distribute or dispense the specimen and/or the reagent to the above-described chambers, that is, from one chamber to another chamber. The dispensing tip 20 may have a tubular shape, and the diameter of the dispensing tip 20 may gradually decrease toward a terminal end thereof, so that the terminal end thereof may have a sharp shape. The dispensing tip 20 described above may be used together with a device that does not have a separate reagent-supplying apparatus and decontamination means, thereby simplifying the operation of the device.

The dispensing tip 20 and the washing tip 30 may be equipped in each of a plurality of cuvettes used in the apparatus according to this embodiment, and thus the tips may be used separately for different cuvettes, thereby preventing contamination. In the case of a conventional automation device using a syringe needle made of a metal material, it is required to provide an apparatus for performing washing in order to prevent contamination. Accordingly, there are problems in that the volume thereof is increased due to the constitution of separate apparatuses, a separate process for washing the apparatuses is required, and inspection costs are increased.

The dispensing tip 20 is seated in the dispensing-tip-insertion hole 21 in the cuvette 10 while being fitted thereinto, and is then fastened to the sampling arm 556, as will be described later, when the inspection process is started, thus enabling suction or discharging for distribution or dispensing of the specimen or the reagent to the chambers together with the pump unit 506. Further, in order to perform the reaction in a second or third cuvette when the reaction occurs in a first cuvette during the inspection process, the dispensing tip used in the first cuvette may be temporarily stored in the insertion hole 21. Accordingly, it is possible to use only one tip for one cuvette until the end of the inspection, without replacing the tip at an intermediate stage. Thus, there are merits in that convenience is secured and a reaction time is reduced. This will be described in more detail in the operation process of the apparatus of the present invention.

The washing tip 30 has a tubular shape having a predetermined height and width and is a member having a sealed bottom end, and an introduction hole having a predetermined depth and inner diameter is formed in an upper part thereof. The washing tip 30 may include a non-magnetic material so as to transmit magnetism, and may also include a flexible material so as to facilitate fixing to the washing arm and separation from the washing arm. Further, the washing tip 30 is seated in the washing-tip-insertion hole 21 in the cuvette 10 while being fitted thereinto, and is then fastened to a straw arm 554 when the inspection process is started, thus performing washing, as will be described later. Further, in order to perform the reaction in the second or third cuvette when the reaction occurs in the first cuvette during the inspection process, the washing tip used in the first cuvette may be stored in the insertion hole 31. Accordingly, it is possible to use only one tip for one cuvette. Thus, there are merits in that convenience is secured and a reaction time is reduced. This will be described in more detail in the operation process of the apparatus of the present invention.

In the embodiment, three cuvettes according to this embodiment are used and are optimized for performing three types of assays. For example, with respect to the same biological specimen, examples of three different analytes may include FT4 (free thyroxine), TSH (thyroid stimulating hormone), and T3 (triiodothyronine) for diagnosis pertaining to the thyroid gland, and hCG (chorionic gonadotropin), E3 (estriol), and AFP (alpha fetoprotein) for congenital anomaly tests.

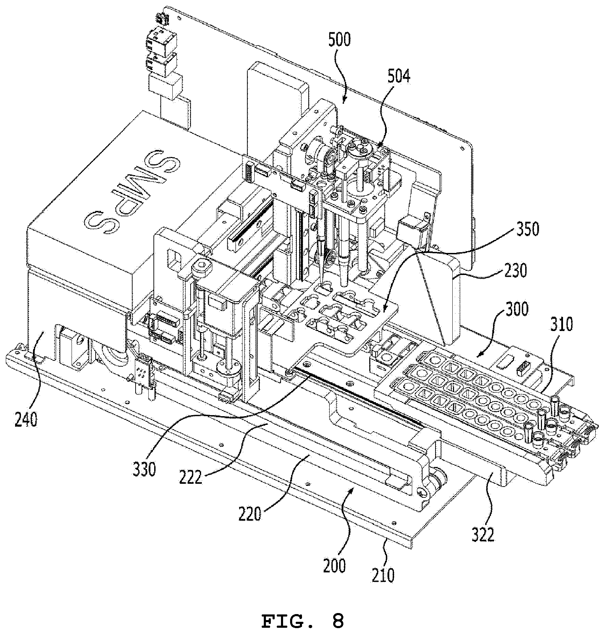

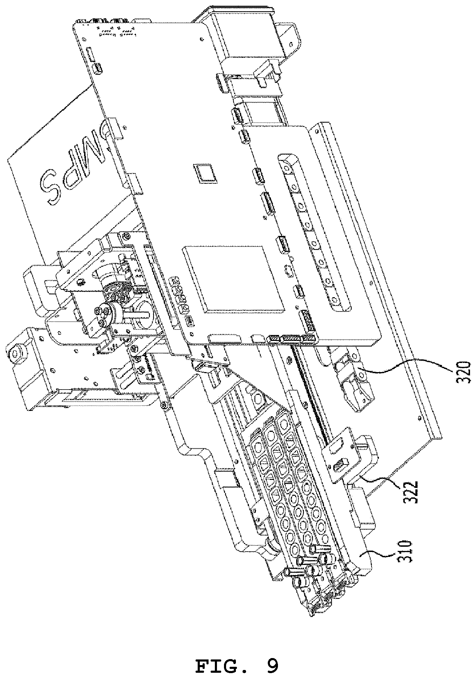

FIGS. 8 and 9 illustrate a liquid-phase immunoassay apparatus 1 according to one embodiment of the present invention, viewed from another direction and omitting the housing 100. As shown, the liquid immunoassay apparatus 1 includes a housing 100, a frame 200, a cuvette module 300, an optical readout module 400, and a dispenser module 500.

The housing 100 constitutes the entire exterior of the automated liquid-phase immunoassay apparatus 1, and functions to block the inflow of foreign materials into the apparatus. The housing 100 may be provided with various input parts for operation and a display unit 110 for output. Further, the housing 100 is provided with an inlet-and-outlet port 120 through which the cuvette 10 is inserted. When the cuvette 10 is inserted into the housing 100 through the inlet-and-outlet port 120, the housing 100 may block the inflow of foreign materials into the chamber included in the cuvette 10, thereby enabling more accurate inspection of specimens.

The frame 200 may be provided in the housing 100 so as to fix the cuvette module 300, the optical reading module 400, and the dispenser module 500. The frame 200 may include a lower frame 210, a first side frame 220, a second side frame 230, and a rear frame 240.

The lower frame 210 is positioned at the lower part of the automated liquid-phase immunoassay apparatus 1. The lower frame 210 may have a plate-like structure having a predetermined area.

The first side frame 220 and the second side frame 230 may be positioned on the left and right sides of the lower frame 210, respectively, and may be constituted so as to have a predetermined height. In addition, the first side frame 220 and the second side frame 230 may have respective guide spaces 222 and 232 for guiding displacement of the holder 310 in forward and backward directions.

The cuvette module 300 is provided in the housing 100 and is an apparatus for receiving the cuvette 10 and moving the received cuvette 10 in forward and backward directions. The cuvette module 300 may include a holder 310, a holder-driving unit 320, a holder guide part 330, and a remover module 340.

The holder-driving unit 320 is a member which exerts forward and backward force on the holder 310. The holder-driving unit 320 may include a movable body 322, to which the holder 310 is fixed, a driving motor, and a predetermined transmitting member for transmitting the power of the driving motor to the movable body 322.

The holder guide part 330 is provided so as to guide the displacement of the holder 310 in forward and backward directions. The holder guide part 330 may include a predetermined guide rail extending in forward and backward directions and a predetermined guide unit, which is connected to the guide rail so as to be movable forwards and backwards along the guide rail and which is also connected to the movable body 322.

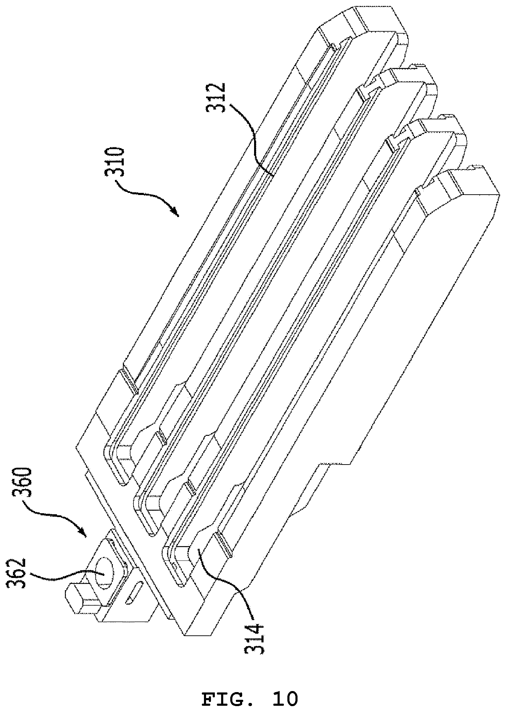

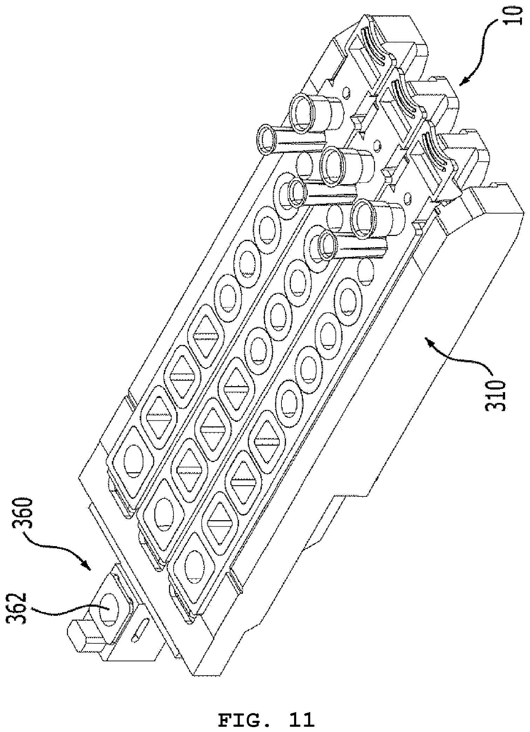

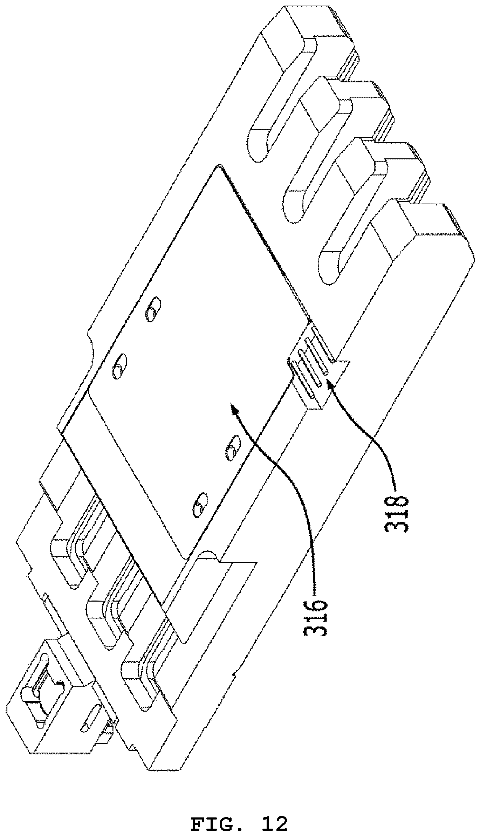

FIG. 10 is a schematic diagram of the holder in a liquid-phase immunoassay apparatus 310 according to an embodiment of the present invention, FIG. 11 illustrates a state in which the cuvette 10 is mounted on the holder shown in FIG. 10, and FIG. 12 illustrates the lower part of the holder shown in FIG. 10.

The holder 310 is a member in which the cuvette 10 may be seated. For example, the holder 310 may be positioned on the lower frame 210 and at the rear of the inlet-and-outlet port 120 in the housing 100. Thus, the cuvette 10 may be fitted into the holder 310 through the inlet-and-outlet port 120. Meanwhile, the holder 310 may have a slot-shaped equipment channel 312 into which one or more cuvettes 10 are inserted so as to be equipped therein. The equipment channel 312 may have a constitution which is elongated in forward and backward directions and opened forwards.

An inspection hole 314 is formed at a rear end of the equipment channel 312. The inspection hole 314 is a portion formed through the equipment channel in upward and downward directions. Therefore, when the cuvette 10 is received and then equipped in the equipment channel 312 of the holder 310, the lower part of a portion of the rear part of the holder 310 is exposed through the inspection hole 314 in a downward direction. To be specific, the lower part of the detection chamber 16 positioned at the rear of the cuvette 10 may be exposed through the inspection hole 314 in a downward direction.

Further, a plurality of equipment channels 312 may be formed in the holder 310 so that the cuvettes 10 are inserted into the respective equipment channels 312 and inspection of a plurality of cuvettes 10 is performed. The plurality of equipment channels 312 may be positioned in a side-by-side arrangement in a single holder 310.

The lower part of the holder 310 is provided with a heat plate 316 and a heat plate power supply 318. This is to automatically control the temperatures of the cuvette and the reactants contained in the cuvette so as to maintain a constant temperature during the reaction, which ensures precision and accuracy of the inspection depending on the characteristics of the biological specimen, which is sensitive to temperature.

The heat plate 316 functions to heat the holder 310 so that the cuvette 10 and the specimen and the reactants contained in the cuvette are heated to a predetermined temperature and maintained at a specific temperature due to convection. The temperature is automatically controlled using the built-in program. A temperature sensor is employed for automatic control, and in the embodiment, the temperature sensor is used in the holder, the heat plate, and the apparatus. Since the temperature of the interior of the apparatus affects an optical system, the temperature sensor of the apparatus is used for temperature control of the interior of the apparatus. The temperature sensor of the heat plate controls the temperature of the heat plate, and the temperature sensor of the holder measures the temperature of the holder to thus control the heat plate in a feedback manner.

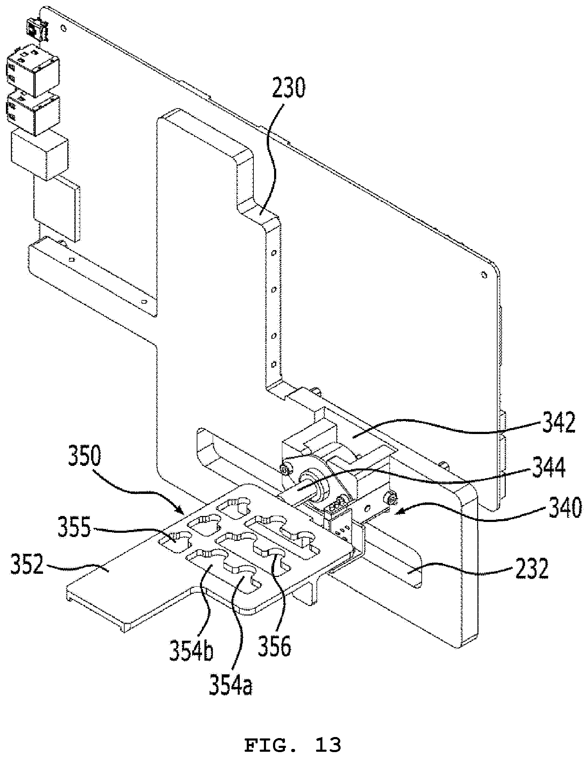

FIG. 13 is a schematic diagram of the remover module 340 in the liquid-phase immunoassay apparatus according to an embodiment of the present invention.

The remover module 340 is a member for dispensing/mixing the reagents in different cuvettes for the immunoreaction time (incubation) after the use of the dispensing tip 20 and the washing tip 30 during an immunity inspection, or a member for removing the tip after the reaction is finished in each cuvette.

The remover module 340 may include a predetermined driving apparatus 342, which is fixed to the second side frame 230, and a predetermined remover plate 350, which is displaced using the driving apparatus 342. The driving apparatus 342 and the remover plate 350 may be connected through a predetermined shaft 344.

The remover plate 350 is located between the holder 310 and the dispenser module 500, as shown in FIG. 8. The remover plate 350 has a plate body 352, and the plate body 352 has a remover line in which three remover holes 354a, 354b, and 355 are formed in a line. The number of remover lines that are formed corresponds to the number of equipment channels 312 formed in the holder 310. The two remover holes 354a and 354b of the remover line are formed so that the holes are connected to each other and are located between the holder 310 and the dispenser module 500. Thus, a punching arm 552 and a straw arm 554, which will be described later, pass therethrough. A sampling arm 556 passes through one remover hole 355 formed alone in the remover line.

Each of the remover holes 354a, 354b, and 355 may have a depression part 356 depressed to one side. Accordingly, in the state in which the dispensing tip 20 fastened to the sampling arm 556 and the washing tip 30 fastened to the straw arm 554 are located in corresponding remover holes 354a, 354b, and 355, the remover plate 350 is displaced to the left in a horizontal direction so that the sampling arm 556 is located at the depression part 356. A portion of the upper end of the dispensing tip 20 is located below the depression part of the plate. When the sampling arm or the straw arm is moved in an upward direction, force may be applied to a portion of the upper end of the dispensing tip 20 fastened to the sampling arm 556 or the washing tip 30 fastened to the straw arm 554, thus removing the tips from the respective arms.

The remover hole 355 is wider than the area of the top of the dispensing tip 20 or the washing tip 30, so that the sampling arm 556 with the dispensing tip 20 or the straw arm 554 with the washing tip 30 can pass through the remover hole 355. Preferably, the depression part 356 is larger than the radius of the sampling arm 556 or the straw arm 554 so that the sampling arm 556 or the straw arm 554 can rest in the depression part 356. Preferably, the depression part 356 is smaller than the area of the top of the dispensing tip 20 or the washing tip 30 so that the top of the dispensing tip 20 or the washing tip 30 can rest on the protruding portion. However, the shape is irrelevant as long as the dispensing tip 20 or the washing tip 30 can be separated from the sampling arm or the straw arm.

The reaction occurring in the cuvette 10 used in the apparatus according to this embodiment requires a minimum of two or more incubation processes from start to detection. The provision of the remover module 340 in the apparatus according to this embodiment has a merit in that the reactions in the different cuvettes equipped in the different equipment channels 312 are prepared for an incubation time even when only one dispensing tip and one washing tip are used in one cuvette, as will be described later.

To be specific, in order to dispense/mix the reagents in the cuvette provided in the second equipment channel for a first incubation time, for which an immunoreaction occurs in the cuvette equipped in the first equipment channel 312, the dispensing tip 20 and the washing tip 30 which have been used in the first channel are temporarily stored at locations 21 and 32 corresponding to the first cuvette. After the lapse of the first incubation time, the dispensing tip 20 and the washing tip 30, which are temporarily stored, may be reused. That is, when the remover module 340 is not provided, the dispensing tip 20 or the washing tip 30, which has been used once in the first equipment channel, is not capable of being reused, but must be removed, and after the first incubation has elapsed, a new tip must be equipped, followed by a subsequent process. Accordingly, at least two dispensing tips 20 and at least two washing tips 30 are required per cuvette provided in the equipment channel. However, according to the present invention, since the remover module 340 is provided, there is a merit in that it is possible to perform the inspection process using only one dispensing tip 20 and one washing tip 30 for each cuvette.

As shown in FIG. 10, the immunoassay apparatus 1 may include a standard block 360. The standard block 360 may be fixed to the holder 310 so as to be integrally displaced together with the holder 310, and may be located at the rear of the holder 310. Preferably, the standard block 360 may be located at the rear of at least one inspection hole 314, among the above-described inspection holes 314.

The standard block 360 has a predetermined optical hole 362 formed in upward and downward directions therethrough, and the optical hole 362 may be provided with a predetermined optical means that is optically detected or captured.

In the embodiment, the standard block 360 includes the optical means. In the embodiment, the optical means included in the standard block 360 includes a standard material for fluorescence measurement, having a predetermined fluorescence value, mounted thereon. The standard material for fluorescence measurement may be a material having appropriate excitation and emission wavelengths depending on the type of fluorescence detected in the material resulting from the reaction.

In another embodiment, the optical means included in the standard block 360 includes a standard material for absorbance measurement, having a visible color, mounted thereon. The standard material for absorbance measurement may be selected appropriately according to the absorbance region of the visible color detected in the material resulting from the reaction. In the embodiment, a glass plate, a plastic plate, a gel, and a suitable liquid solution are used, without limitation thereto.

In the optical assay, when the fluorescence or absorbance value of the material resulting from the reaction is measured after completion of the reaction, the standard fluorescence or absorbance provided using the standard block 360 is first scanned, and the signal value of the material resulting from the reaction is measured, which is represented by a ratio. This is to eliminate deviations between the instruments. The standard material is used to calculate the ratio to the measured value, and the ratio is compared to built-in data using a master calibration graph to thus accurately calculate the concentration of the analyte in the specimen.

When measuring the fluorescent or absorbance signal, the absolute values of the fluorescence values between the devices are generally different from each other. Accordingly, when the concentration is calculated using the absolute value of fluorescence, there is a problem in that an error may occur depending on the type of device. Therefore, when the ratio to the measured value is used with the standard material of the standard block, as in the present invention, the error of the measured value between the devices is reduced, and accuracy and reproducibility are improved.

In yet another embodiment according to the present invention, the apparatus may not include the standard block 360, or the standard block may not be used even if the apparatus includes the standard block 360. For example, when the signal detected in the material resulting from the reaction is chemiluminescent, the apparatus may not include the standard block, or the standard block may not be used, even if the apparatus includes the standard block. In this case, the apparatus may include a photodetector such as a PMT and an avalanche photodiode, and may be provided with a shutter embodied in hardware or software as a means for measuring the quantity of light for a predetermined constant time in order to measure the relative quantity of light. Accordingly, deviations in the detected signals between the apparatuses may be compared, followed by correction.

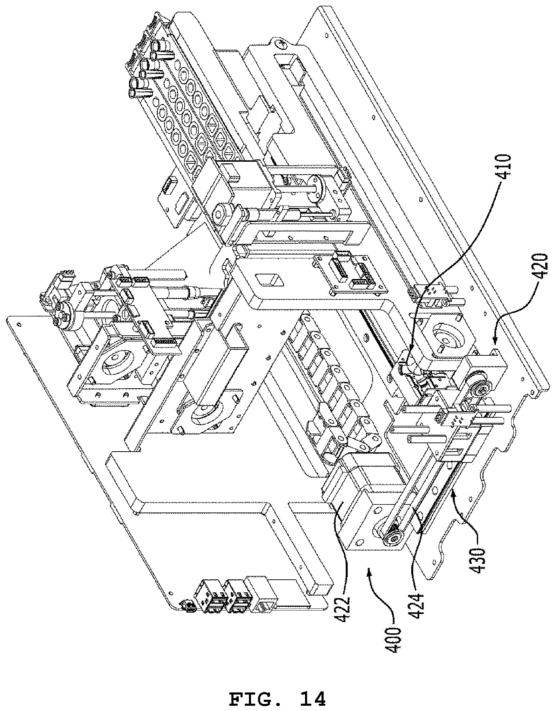

FIG. 14 is a view of the liquid-phase immunoassay apparatus 1 shown in FIG. 8 with the rear frame omitted.

When the holder-driving unit 320 is operated, the holder 310 may be displaced in forward and backward directions. When the holder 310 is moved in a backward direction by a predetermined distance, the standard block 360 fixed to the holder 310 is located on an optical reader 410, as will be described later. Accordingly, the fluorescent signal of the standard block 360 may be captured using the optical reader 410.

Further, when the holder 310 is moved to the rear end, the lower part of the rear part of the holder 310 is located on an optical reading module 400, as will be described later. Therefore, when the holder 310 is moved to the rear end while the cuvette 10 is equipped in the equipment channel 312 of the holder 310, the lower part of the detection chamber 16 positioned at the rear of the cuvette 10 may be exposed to the optical reading module 400 through the inspection hole 314.

Since the displacement of the holder 310 is guided by the holder guide part 330, the displacement may be stably performed without rocking. In particular, a pulley-belt-type holder-driving unit 320 may be provided so as to prevent the occurrence of vibration and the inflow of foreign materials caused by friction generated during movement, thereby achieving more accurate inspection compared to a gear type.

The optical reading module 400 is provided for measuring the signal of the material resulting from the reaction in the cuvette 10. Preferably, the optical reading module 400 may include an optical reader 410, a reader-driving unit 420, and a reader guide part 430. The optical assay is performed by the optical reading module 400. The optical assay may include measurement of the fluorescent signal, the visible color, or the chemiluminescence of the material resulting from the reaction, and each of the signals may be defined with reference to the above.

The optical reader 410 is located below the holder 310 when the holder 310 is moved to the rear end. Therefore, when the holder 310 is moved backwards while the cuvette 10 is received in the holder 310, the detection chamber 16 of the cuvette 10 is located on the optical reader 410. Accordingly, the measurement of the fluorescence value for the material resulting from the reaction in the detection chamber 16 may be performed using the optical reader 410.

The optical reader 410 may read the signal of the material resulting from the reaction in the detection chamber 16 of the cuvette 10 to thus enable the qualitative and/or quantitative assay of a specific target analyte contained in the specimen.

In the embodiment, the optical reader 410 of the optical reading module detects a fluorescent signal. The optical reader 410 may radiate light having a specific wavelength depending on the type of the fluorescent material used in the detection of the analyte, and may read the emitted light.

The optical reader 410 may be provided with a light source 610 (i.e., a light emitting element) whose output is capable of being adjusted and which is capable of sufficiently exciting the fluorescent material for measurement of the fluorescent signal, that is, a predetermined light-emitting device. Examples of the light-emitting device include a Xenon lamp, a UV laser, or an LED (light-emitting diode).

In particular, as mentioned above, by irradiating the standard block 360 with light before measuring the fluorescence value, the gain can be automatically adjusted based on the amount of fluorescence captured, so that the output of the light-emitting element can be adjusted to a predetermined value, enabling accurate calculation of the concentration.

The optical reader 410 may have two or more light sources, and the light sources may generate light having different wavelengths. In addition, the fluorescences of different wavelengths may be measured separately. Therefore, the application range thereof to the diagnostic test method may be widened and the sensitivity thereof may be further improved.

The optical reader 410 may have a barcode scanner function, so that when a predetermined barcode is provided in the cuvette 10, a predetermined signal and information exchanges may be performed through the corresponding barcode.

In yet another embodiment, the optical reader 410 of the optical reading module serves to measure the absorbance of the visible color of the material resulting from the reaction. According to this embodiment, the absorbance may be measured by radiating light on the material resulting from the reaction depending on the type of material used for detection of the analyte. Meanwhile, the optical reader 410 includes a light source whose output is capable of being adjusted and which is capable of emitting an absorption wavelength region band suitable for absorbance measurement of the visible color. Examples of the light-emitting device may include a lamp, an LED, and a laser including an absorption wavelength band such as a white light source, but without limitation thereto.

In still another embodiment, the optical reader 410 serves to measure a chemiluminescent signal of the material resulting from the reaction. According to this embodiment, the optical reader may detect light emitted depending on the type of chemiluminescent materials used for the detection of the analyte, and includes a lens for trapping light and a photon detector so that the intensity of the light that is emitted is measured for each time.

The reader-driving unit 420 may be provided in the housing 100 and may move the optical reader 410 so that the optical reader 410 is located in any one cuvette 10 of a plurality of cuvettes 10, thereby inspecting the specimen in the corresponding cuvette 10. That is, the reader-driving unit 420 can move the position of the optical reader 410 to align with the inspection hole 314 of the holder 310.

The reader-driving unit 420 may include a predetermined driving motor 422 that enables the optical reader 410 to move leftwards and rightwards, a driven pulley 424, and a predetermined bracket for connecting the driven pulley 424 and the optical reader 410. Therefore, the optical reader 410 may be moved according to the operation of the driving motor 422.

The reader guide part 430 is provided to guide the displacement of the optical reader 410 in leftward and rightward directions. The reader guide part 430 may include a predetermined guide rail and a predetermined guide unit which is guided along the guide rail and fixed to the optical reader. Therefore, the movement of the optical reader in leftward and rightward directions may be unidirectionally guided with accuracy.

As described above, when the holder 310 is moved backwards by a predetermined distance, the standard block 360 at the lower part of the rear part of the holder 310 is located on the optical reader 410 of the optical reading module 400. Accordingly, the optical reading module 400 first senses the fluorescent signal captured in the standard block 360 as a standard fluorescence.

Subsequently, when the holder 310 is moved to the rear end while the cuvette 10 is equipped in the equipment channel 312 of the holder 310, the lower part of the detection chamber 16 positioned at the rear of the cuvette 10 may be exposed to the optical reader 410 through the inspection hole 314, thereby performing optical measurement.

As described above, the ratio of the fluorescent signal captured by the standard block 360 and the fluorescent signal captured in the detection chamber 16 is used for displaying. The optical reading module 400 may have a predetermined algorithm and a predetermined repetitive measurement algorithm that enable the calculation of the concentration of the analyte in the specimen by comparing the above ratio to built-in data using a master calibration graph.

As described above, since the measurement is performed in such a manner that the fluorescence value of the standard fluorescence mounted on the standard block 360 is compared to the fluorescence value of the specimen, measurement may be accurately performed. That is, according to a general conventional technology, there is a difference in fluorescence value between devices, and in order to reduce this difference, it is necessary to perform a calibration process for reducing the difference between instruments during most QC stages. Despite this process, however, it is difficult to completely eliminate the above-described difference due to changes in instruments or reagents. However, in the present invention, since the standard fluorescence mounted on the standard block 360 serves as a reference, the above problem may be solved.

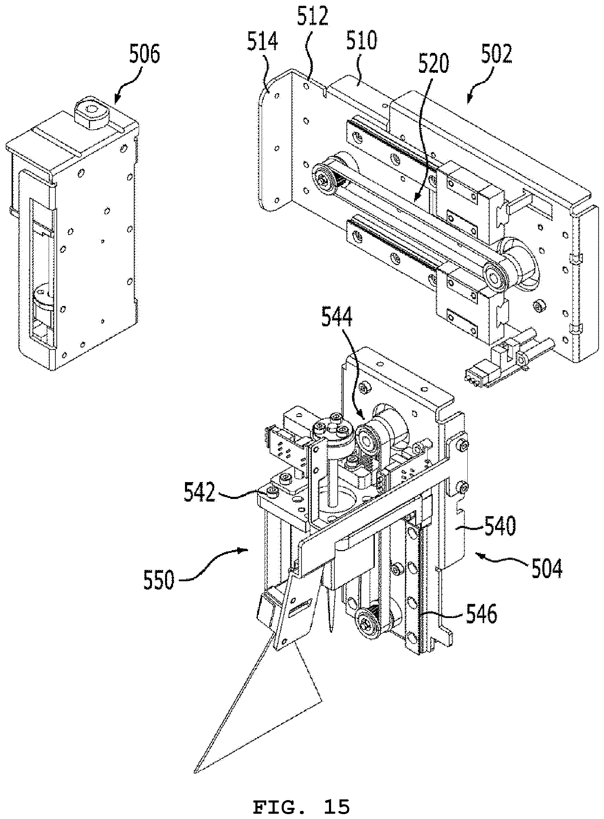

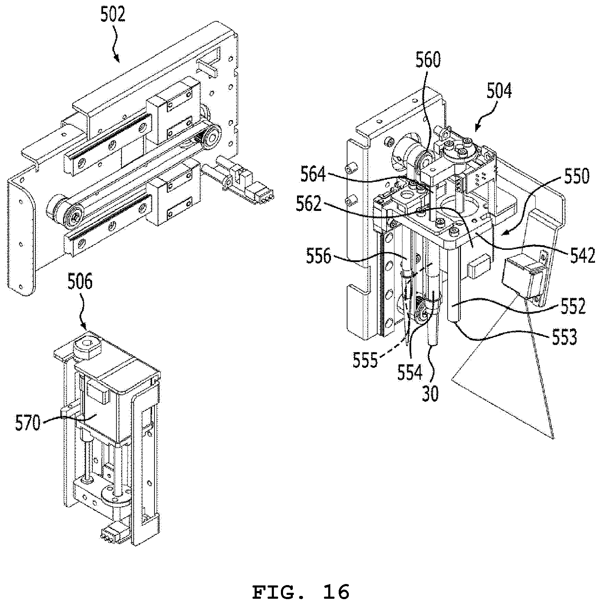

Hereinafter, the dispenser module 500 will be described. FIGS. 15 and 16 are exploded views showing the structure of the dispenser module 500 in the automated liquid-phase immunoassay apparatus 1 according to this embodiment in different directions. The dispenser module 500 is a module provided for distributing, dispensing, and washing specimens, reagents, and/or reactants. As shown, the dispenser module 500 includes a driving unit 502, a dispenser unit 504, and a pump unit 506.

The driving unit 502 serves to horizontally move the dispenser unit 504 leftwards and rightwards. Accordingly, the dispenser unit 504 may be horizontally moved by the driving unit 502 so that the dispenser unit 504 is located in a specific chamber on any one cuvette 10 of a plurality of cuvettes 10 located parallel to each other below the driving unit. The driving unit 502 may include a fixing body 510 and a left-and-right horizontal driving unit 520.

The fixing body 510 may have a predetermined area and may be elongated in leftward and rightward directions. The fixed body 510 may include a front body 512, extending in leftward and rightward directions, and a side body 514 which is provided on one side of the front body 512 and to which the pump unit 506 is fixed.

The left-and-right driving unit 520 is a driving means that is positioned on the fixing body 510 and moves the dispenser unit 504, which will be described later, horizontally leftwards and rightwards. The left-and-right driving unit 520 may include a predetermined driving motor for generating power and a predetermined moving bracket capable of being displaced leftwards and rightwards by the driving motor. Further, a predetermined guide means 530 for guiding the displacement of the moving bracket may be provided. In addition, a predetermined driven pulley member that transmits power may be included.

The dispenser unit 504 may include a left-and-right moving body 540, an up-and-down moving body 542, an up-and-down driving unit 544, and an arm unit 550, as shown.

The left-and-right moving body 540 is connected to the left-and-right driving unit 520. As described above, the left-and-right driving unit 520 may include the predetermined moving bracket, and the left-and-right moving body 540 may be connected to the moving bracket so as to be horizontally displaced leftwards and rightwards.

The up-and-down moving body 542 is positioned at the front of the left-and-right moving body 540. The up-and-down moving body may be displaced upwards and downwards by the up-and-down driving unit 544.

The up-and-down driving unit 544 is a driving means that is positioned on the left-and-right moving body 540 and moves the up-and-down moving body 542 in upward and downward directions. Further, the up-and-down driving unit 544 may include a predetermined driving motor for generating power and a predetermined moving bracket capable of being displaced leftwards and rightwards by the driving motor. Further, a predetermined guide means 546 for guiding the displacement of the moving bracket in upward and downward directions may be provided. In addition, a predetermined driven pulley member that transmits power may be included.

The arm unit 550 is a member that is moved upwards and downwards by the up-and-down driving unit 544 and is moved leftwards and rightwards by the driving unit 502. The arm unit 550 may include a punching arm 552, a sampling arm 556, and a straw arm 554, which are connected to the up-and-down moving body 542 and which extend in a downward direction at locations spaced apart from each other in a horizontal direction. Accordingly, the arm unit 550 may include an integrated module in which the punching arm 552, the sampling arm 556, and the straw arm 554 are integrally constituted. The punching arm 552 is provided with a punching tip 553 at a bottom end thereof, and is a member for piercing and opening a sealing cover of the cuvette 10 to thus pierce a sealing portion covering the corresponding chamber of the cuvette 10. The straw arm 554 is open in upward and downward directions to thus have upper and lower hollows 555. The straw arm 554 has an outer diameter suitable to be introduced into the introduction hole in the washing tip 30 so as to be fitted thereinto. The sampling arm 556 is provided so that the dispensing tip 20 is fixed to the bottom end thereof. The sampling arm 556 may have an outer diameter suitable to be introduced into the dispensing tip 20 so as to be fitted thereinto. Preferably, the punching arm 552, the straw arm 554, and the sampling arm 556 may be positioned in a line in forward and backward directions.

The washing unit 560 includes a servomotor 562 and a magnetic beam 564.

The servomotor 562 may be fixed to the up-and-down moving body 542 and connected to the magnetic beam 562 to thus displace the magnetic beam 564 in upward and downward directions. Meanwhile, any predetermined driving apparatus capable of displacing the magnetic beam 564 upwards and downwards may be provided, without limitation to the servomotor 562.

The magnetic beam 564 is constituted in the form of a bar that extends in upward and downward directions, and is positioned in the upper and lower hollows 555 of the straw arm 554. The magnetic beam 554 may be magnetic and may be displaced in upward and downward directions by the servomotor 552 to thus enable mag-extraction for separating unreacted materials using magnetism.

The pump unit 506 is fixed to the side body 514 of the driving unit 502. The pump unit 506 is connected to the sampling arm 556 of the dispenser unit 504 through a predetermined pipe (not shown) so as to provide suction or discharge force when the dispensing tip 20 is inserted into the chamber of the cuvette 10 while being connected to the sampling arm 556. To be specific, the pump unit may provide the suction or discharge force to the dispensing tip 20 when the dispensing tip 20 is introduced into the chamber in the state in which the cuvette 10 is located at a specific site by the cuvette module 300 and the dispensing tip 20 is located on the chamber of the cuvette 10 by the driving unit 502. Preferably, the pump unit 506 may be provided with a motor 570 that enables rotary micro-step controlling so as to accurately adjust the amount of the specimen, the reagent, or the material resulting from the reaction upon suction or discharge thereof with respect to the dispensing tip 20.

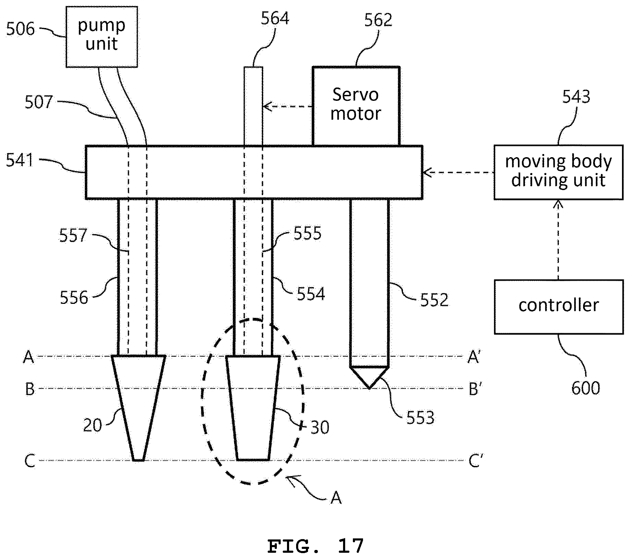

FIG. 17 is a conceptual diagram of a dispenser module according to another embodiment of the present invention.

The dispenser module includes a moving body 541, a moving body driving unit 543, and a controller 600. The controller 600 is capable of controlling the moving body driving unit 543 to move the moving body 541 to a desired position.

A punching arm 552, a straw arm 554, and a sampling arm 556 are fixed on the moving body 541. Accordingly, the punching arm, the straw arm, and the sampling arm move integrally by the movement of the moving body.

The lower part of the punching arm 552 is provided with a punching tip 553. When the punching arm penetrates the seal of the lower cuvette, the straw arm and sampling arm that are fixed to the punching arm and the moving body 541 together and move integrally should not interfere with the lower cuvette. In other words, the length B between the lower part of the moving body and the lower part of the punching arm 552 has to be longer than the lengths A of the straw arm and the sampling arm. Even if the punching arm moves downwards to the maximum to penetrate the seal of the cuvette, the proper length can be set so that the straw arm and sampling arm may not reach the cuvette.

In the case that the straw arm 554 with the washing tip 30 or the sampling arm 556 with the dispensing tip 20 works with the cuvette, the punching arm 552 should not interfere with the cuvette below. Therefore, the length B between the lower part of the moving body and the lower part of the punching arm 552 has to be shorter than the length C between the lower part of the moving body and the end of the washing tip 30 mounted on the straw arm or the end of the dispensing tip 20 mounted on the sampling arm. That is, the heights of the washing tip 30 and the dispensing tip 20 should be larger than the sum of the length of the punching arm and the depth of each chamber in the cuvette. Each tip can be set to a suitable length in consideration of the mounting location of each arm and the smooth operation distance in each chamber.

The sampling arm 556 can be mounted by fixing the dispensing tip 20 at its lower part. A sampling hollow 557 is provided to penetrate the inside of the sampling arm vertically. The hollow of the sampling arm is connected to the pump unit 506 through a pipe 507. The pump unit 506 can supply a suction force and a discharge force to the dispensing tip 20 through the pipe and the hollow of the sampling arm.

The straw arm 554 can be mounted by fixing the washing tip 30 to its lower part. Upper and lower hollows 555 are provided to penetrate the inside of the straw arm vertically. A magnetic beam 564 is located in the hollow of the straw arm so as to move up and down. A servomotor 562 is provided to move the magnetic beam up and down. It is preferable to fix the servomotor 562 to the moving body so that the magnetic beam can perform relative motion with respect to the straw arm fixed to the moving body.

The connection between the servomotor and the magnetic beam can be achieved by means of a linear actuator using a ball screw, etc., a reducer using gear coupling, a rack and pinion, and the like.

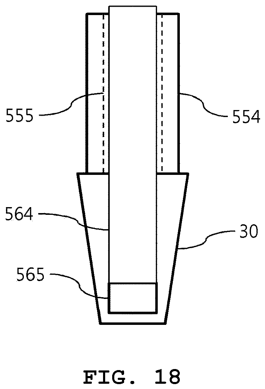

FIG. 18 is a detailed view of part "A" shown in FIG. 17.

A magnetic beam 564 is placed in the upper and lower hollows 555 of the straw arm 554. The magnetic beam 564 may be provided with a permanent magnet 565 at a lower part of the opposite end of a portion connected to the servomotor 562. The permanent magnet 565 preferably has the same cross-sectional area as that of the attached magnetic beam. If the magnetic beam is cylindrical, a cylindrical permanent magnet with the same diameter can be used. When the magnetic beam 564 is moved down by the servomotor 562, a permanent magnet can be disposed inside the washing tip 30 into which the straw arm 554 is inserted.

The size of the permanent magnet 565 can be smaller than that of the chamber of the cuvette, and the permanent magnet 565 can be selected to use in various shapes such as round, square, and oval depending on the purpose.

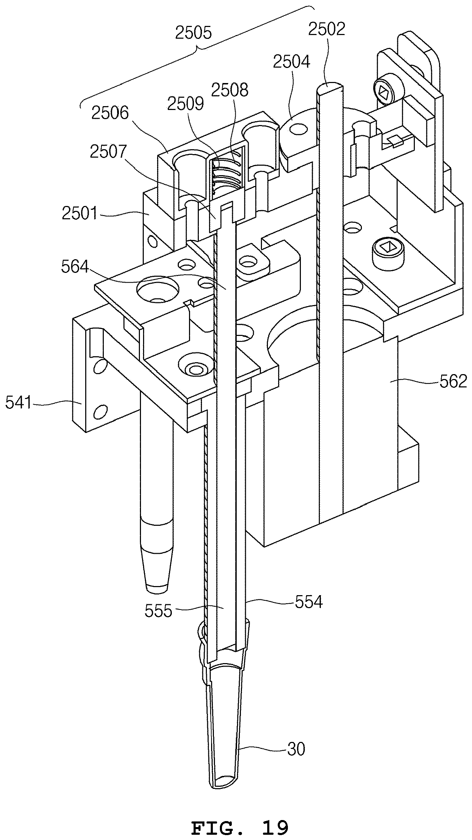

FIG. 19 is a partial cross-sectional view of a dispenser module according to another embodiment of the present invention. As shown in FIG. 19, a servomotor 562 and a straw arm 554 are installed in a moving body 541. The driving force of the servomotor 562 is transmitted to the magnetic beam 564 via the motion conversion member 2504, the connection member 2501, and the socket member 2505.

The driving force of the servomotor 562 is implemented by a rotational motion of the shaft 2502. The rotational motion of the shaft 2502 is converted into linear motion by the motion conversion member 2504. The motion conversion member 2504 is screwed to the shaft 2502 and fixed to the connection member 2501 so as not to rotate. Therefore, when the shaft 2502 rotates, the motion conversion member 2504 and the connection member 2501 move upwards or downwards according to the rotation direction of the shaft 2502. Though the motion conversion member can be implemented by a cam, a crank, or the like, a screw coupling method is advantageous because the configuration is simple and compact.

A socket member 2505 is fixed to the connection member 2501. The socket member 2505 includes a mounting part 2506, a cap 2507, and a spring 2508. The mounting part 2506 has a hole 2509 formed downward. A spring 2508 is mounted in the hole 2509, and a cap 2507 is installed so that the upper side of it presses the spring 2508 while sliding in the hole 2509. The magnetic beam 564 is screwed to the bottom of the cap 2507 so as to be fixed to the socket member 2505.

When power is applied to the servomotor 562 and the shaft 2502 rotates, the motion conversion member 2504 moves upwards or downwards according to the direction of rotation. Since the motion conversion member 2504 is fixed to the connection member 2501, the connection member 2501 also moves upwards or downwards as the motion conversion member 2504 moves upwards or downwards. Since the socket member 2505 is fixed to the connection member 2501, the socket member 2505 also moves upwards or downwards, as the connection member 2501 moves upwards or downwards. As the socket member 2505 moves upwards or downwards, the magnetic beam 564 mounted on the socket member 2505 also moves upwards or downwards. Since the servomotor 562 and the straw arm 554 are installed in the moving body 541, the magnetic beam 564 moves upwards or downwards in the hollow 555 formed inside the straw arm 554. A permanent magnet (not shown) is attached to the end of the magnetic beam 564, and a washing tip 30 is connected to the end of the straw arm 554. Therefore, when the shaft 2502 of the servomotor 562 rotates, the permanent magnet moves upwards or downwards inside the washing tip 30 according to the rotation direction of the shaft 2502.

Since the spring 2508 pushes the magnetic beam 564 in the direction of the washing tip 30 via the cap 2507, the permanent magnet attached to the end of the magnetic beam 564 adheres closely to the washing tip 30. The permanent magnet can be brought into close contact with the washing tip 30 only by the rotation of the servomotor 562. However, if the magnetic beam 564 moves downwards excessively, the washing tip 30 gets separated from the straw arm 554, because the servomotor 562 is not precisely controlled. So, there is a risk of ruining the entire analysis process.

In this embodiment, the spring 2508 is located on the socket member 2505 for mounting the magnetic beam 564, but may be located elsewhere on the path through which the driving force of the servomotor 562 is transmitted to the magnetic beam 564. In addition, a polymer having elasticity may be used instead of the spring 2508.

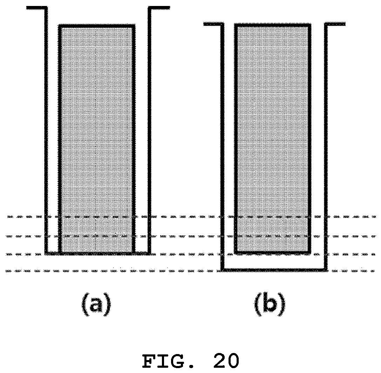

FIG. 20 is a view explaining the positional relationship between the washing tip 30 and the permanent magnet attached to the end of the magnetic beam 564 in the dispenser module shown in FIG. 19. FIG. 20A shows a state in which the permanent magnet is in close contact with the washing tip 30 without gap by the spring 2508, and FIG. 20B shows that there is a gap between the permanent magnet and the washing tip 30 when the magnetic beam 564 is moved downwards only by the rotation of the servomotor 562 without any elastic body.

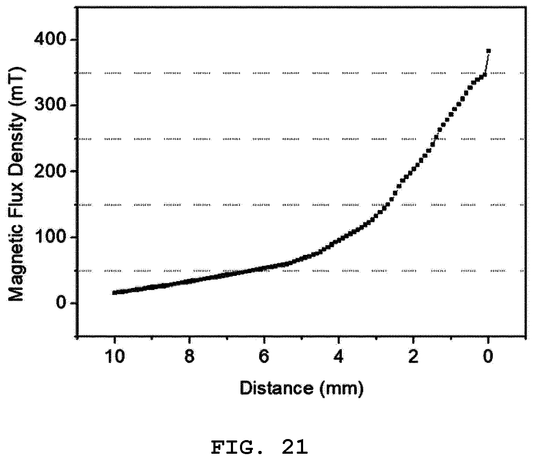

Fig. 21 is a diagram explaining the relationship between the distance from the permanent magnet and the magnetic flux density. The magnetic flux density is inversely proportional to the square of the distance. As shown in FIG. 20A, when the permanent magnet is in close contact with the washing tip 30 without a gap, the magnetic flux density at the lower end of the washing tip 30 is constant and has a large value compared to FIG. 20B, and therefore the magnetic beads are also attached to the lower end of the washing tip 30 in a constant and large amount in proportion to the magnetic flux density.

According to the present invention, it is possible to minimize the loss of the collected magnetic bead complex along with the washing solution in the driving to move to the next step after collecting the magnetic beads. Therefore, the reproducibility of the immunoreaction is increased, and the coefficient of variation (CV) is reduced.

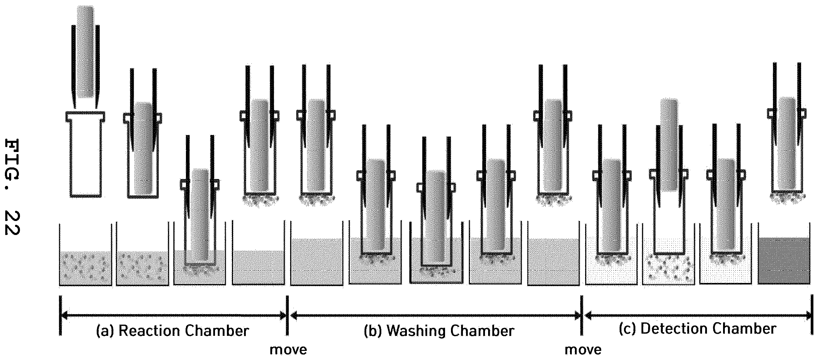

FIG. 22 is a diagram explaining the positional relationship between the washing tip 30 and the magnetic beam 564 in the immunoreaction analysis process according to an embodiment of the present invention. As shown in FIG. 22A, in the reaction chamber 14, in a state in which the magnetic beam 564 is moved downwards to the end of the hollow part of the washing tip 30, the magnetic beads (or magnetic bead immuno-complex) in the reaction chamber 14 are collected and moved to the washing chamber 15. As shown in FIG. 22B, in the washing chamber 15, the magnetic beam 564 stays as moved down to the end of the hollow part of the washing tip 30. In a state where the magnetic beads are attached to the surface of the washing tip 30, the washing tip 30 is moved up and down n times repeatedly to wash the non-specific biological sample with the washing solution. As shown in FIG. 22C, in the detection chamber 16, the magnetic beam 564 is moved upwards from the end of the hollow part of the washing tip 30 to disperse the magnetic beads attached to the surface of the washing tip 30 into the substrate in the detection chamber 16. The enzyme of the magnetic immuno-complex reacts with the substrate in the detection chamber 16. When a predetermined time elapses after the magnetic beads are dispersed, the magnetic beam 564 is moved down to the end of the hollow part of the washing tip 30 to collect the magnetic beads on the surface of the washing tip 30 again. After the washing tip 30 is moved to the outside of the detection chamber 16 in a state where the magnetic beads are collected, an optical inspection is performed on the detection chamber 16.

As in the present embodiment, when the washing tip 30 is moved out of the detection chamber 16 in a state in which magnetic beads are collected again in the detection chamber 16, the time for optical inspection increases, but the magnetic bead immuno-complex and the bias signal due to the reaction of the substrate is reduced.

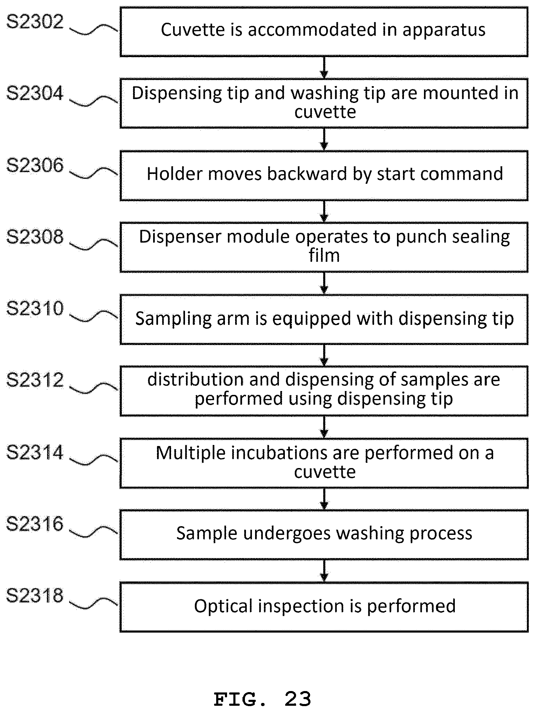

FIG. 23 is an overall flowchart of a liquid-phase immunoassay method according to an embodiment of the present invention.

First, the cuvette 10 is accommodated in the equipment channel 312 of the holder 310 of the immune response analysis apparatus 1 (S2302). At this time, the dispensing tip 20 and the washing tip 30 are mounted in the dispensing-tip-insertion hole 21 and the washing-tip-insertion hole 31 formed in the cuvette (S2304). The dispensing tip 20 and the washing tip 30 can be applied either before or after accommodating the cuvette 10 in the equipment channel 312. Subsequently, the holder 310 moves backward by the start command of the apparatus (S2306).

Subsequently, the dispenser module 500 operates to punch the sealing film (not shown) of the cuvette 10 to open (S2308). In the punching process, a punching arm 552 is used. Describing this punching process, first, the punching arm 552 is positioned over the cuvette 10 by a driving unit, and then the punching arm 552 is moved up and down by the up-and-down driving unit 544 so to punch the sealing film of the cuvette 10. During this process, the cuvette module 300 operates and the cuvette 10 moves forward or backward, so that punching can be performed in a plurality of chambers provided in the cuvette 10.

When punching is completed, the cuvette module 300 and the dispenser module 500 operate so that the sampling arm 556 is located on the dispensing tip 20 fixed to the cuvette 10. Subsequently, the sampling arm 556 is moved downwards to insert the lower part of the sampling arm 556 into the dispensing tip 20 to fix them (S2310). Thereafter, distribution and dispensing of samples and/or reagents are performed using the dispensing tip 20 (S2312).

A detailed description of the dispensing process is as follows. First, the dispensing tip 20 is put into the sample solution by moving the moving body 541 to which the sampling arm 556 is fixed. Then, the pump unit 506 connected to the hollow of the sampling arm is operated to apply a suction force to the dispensing tip 20 so as to collect a sample from the sample chamber. Next, the moving body driver 543 is driven to move the sampling arm fixed to the moving body to the reaction chamber. At this time, the sample in the dispensing tip 20 attached to the sampling arm is also moved to the reaction chamber. That is, the collected sample may be moved to the reaction chamber. Then, the pump unit 506 is operated to apply a dispensing force to the dispensing tip 20 to discharge the sample into the reaction chamber so as to complete dispensing.

In this process, as in the previous punching process, the cuvette 10 can be moved forward or backward by the cuvette module 300, and the dispensing tip 20 can be moved upward and downward by the up-and-down driving unit 544. At the same time, the pump unit 506 operates to perform distribution and dispensing by the dispensing tip 20. In addition, the operation of the pump unit 506 allows mixing of samples and/or reagents during the distributing and dispensing process, and allows desired reactions to occur in the reaction chamber 14 of the cuvette.

As such, the reaction process occurring in the cuvette 10 includes a plurality of steps, and requires at least two incubation times per cuvette (S2314). The incubation may be performed by applying power to the heat plate 316 of the holder 310 on which the sample is mounted so as to keep the sample dispensed in the reaction chamber at a constant temperature.

Therefore, during the first incubation time, in order to start the reaction of the second cuvette, the dispensing tip 20 used in the first cuvette is removed by the remover plate 350 and is located in the dispensing-tip-insertion hole 21 of the first cuvette. After completion of the first incubation time, the dispensing tip 20 is reused for the reaction of the next step of the first cuvette.

After the incubation is complete, the sample undergoes a washing process (S2316). After the washing process, the sample containing the magnetic beads from which impurities are removed is moved to the detection chamber and used for analysis through an optical inspection process (S2318).

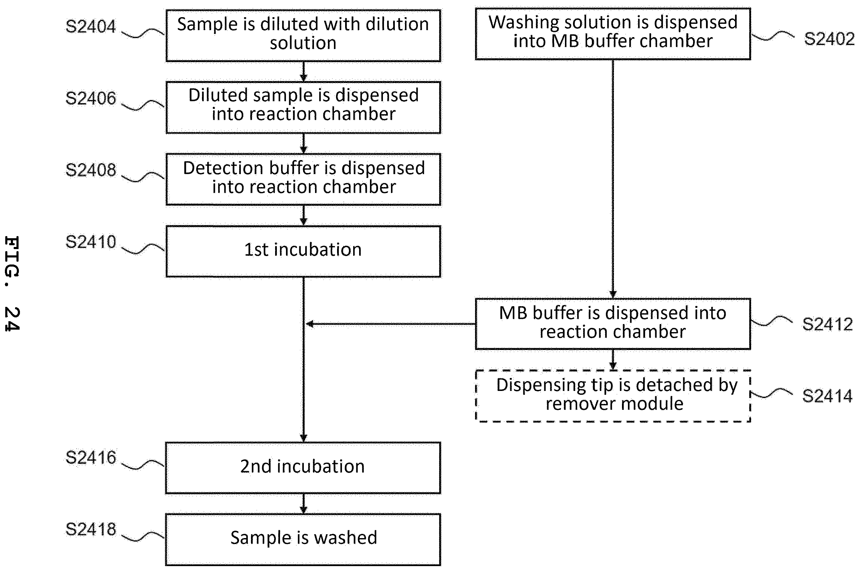

FIG. 24 is a flowchart of a sample dispensing process according to an embodiment of the present invention.

First, after recognizing the barcode, the seals of the cuvette 10 are punched with the punching arm 552 to open them. Subsequently, the sampling arm 556 is inserted into the dispensing tip 20 so that the dispensing tip 20 may be fixed to the sampling arm 556. And then, a predetermined volume of washing solution is collected from the first washing chamber 15a and dispensed into the MB buffer chamber 13a (S2402).

Subsequently, a predetermined dilution solution is collected from the dilution buffer chamber 13c, and dispensed into the sample chamber 12 (S2404), so that a mixing process (three times) may be performed. Subsequently, a predetermined volume of diluted sample is collected and dispensed into the reaction chamber 14 (S2406). And then, after mixing the chamber 13b filled with the detection buffer, a predetermined volume of solution is collected and dispensed to the reaction chamber 14 (S2408) so as to be mixed (three times). Subsequently, a first incubation process is performed at a specific temperature for a predetermined time (S2410). Next, after mixing the MB buffer chamber 13a, a predetermined volume of solution in the MB chamber 13a is collected and dispensed to the reaction chamber 14 (S2412) so as to be mixed. Subsequently, the dispensing tip 20 is removed using the remover module 340 (S2414), and placed in the dispensing-tip-insertion hole 21 of the cuvette where the reaction is performed. In addition, a second incubation process is performed at a specific temperature for a predetermined time (S2416).

Then, after the lapse of the second incubation time, a washing process is performed (S2418). In the washing process, first, the straw arm 554 is inserted into the washing tip 30, and the magnetic beam 564 is put into the straw arm 554 so that the magnetic beam 564 may be put in the reaction chamber 14 for a predetermined time, and then the magnetic beam 564 is put into the first washing chamber 15a, and moved up and down several times to wash. Subsequently, the magnetic beam 564 is again put into the straw arm 554 and put into the detection chamber 16, and then the washing tip 30 is removed.

Subsequently, after a third incubation process for a predetermined time, an optical measurement process is performed. Results (density, etc.) derived from the optical measurement can be output to a display or a printer.

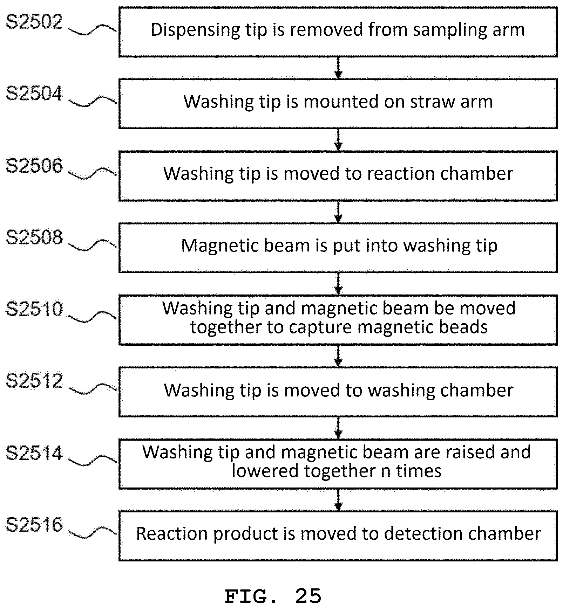

FIG. 25 is a flowchart of a washing process according to an embodiment of the present invention.

First, the washing tip 30 into which the magnetic beam 564 is inserted is put into a sample solution containing magnetic beads to collect the magnetic beads in the sample solution on the surface of the washing tip 30 (S2504 to S2510). Next, the washing tip 30 on the surface of which the magnetic beads are collected is moved to the washing solution in a state where the magnetic beam is inserted, so as to be put into the washing solution (S2512). Next, while maintaining the magnetic beam 564 at the end of the hollow part of the washing tip 30, the washing tip 30 is moved up and down n times (S2514). When the washing is completed, it can be moved to the detection chamber and optical measurement can be performed (S2516).

On the other hand, the step of collecting magnetic beads in the sample solution can be divided as follows. First, the washing tip 30 is fixed to the lower part of the straw arm 554 having a hollow vertically penetrated therein (S2504). Then, the moving body 541 to which the straw arm is fixed is moved down to put the washing tip 30 into the sample solution containing the magnetic beads (S2506). Next, the magnetic beam 564 located in the hollow of the straw arm is inserted into the lower washing tip 30 by driving the servomotor 562 fixed to the moving body (S2508). After that, the moving body driving unit 543 integrally moves the moving body to which the straw arm is fixed and the magnetic beam 564, so that the washing tip into which the magnetic beam 564 is inserted can be moved in the sample solution containing magnetic beads 30 (S2510).

The more detailed description of the above process is as follows. When the distribution, dispensing, and reaction of samples and reagents are completed, the dispensing tip 20 is removed from the sampling arm 556 by the remover plate 350 (S2502). Subsequently, the washing tip 30 is mounted on the straw arm 554 (S2504). The washing tip 30 is put into the reaction chamber 14 (S2506), and then, a magnetic beam 564 is put into the washing tip 30 so that the magnetic beads in the reaction chamber 14 are collected on the surface of the washing tip 30 (S2508). At this time, reactants combined with magnetic beads are collected together. In order to collect the magnetic beads more efficiently, the washing tip 30 and the magnetic beam 564 may be moved together in the sample solution (S2510). In this state, the washing tip 30 is moved to the washing chamber 15 (S2512). In the washing chamber 15, the washing tip 30 is driven to move together with the magnetic beam 564 up and down n times to wash the non-specific biological sample attached to the surface of the washing tip 30 with a washing solution (S2514). The magnetic beam 564 stays at the state where it is moved down to the end of the hollow part of the washing tip 30 during the washing process. The washing tip 30 is moved up and down as many times as the predetermined number of washings. When the sample is finished washing, the reaction product is moved to the detection chamber 16 (S2516).

In this embodiment, non-specific biological samples are removed by driving them to move up and down repeatedly as collected without repeating dispersion and collection of magnetic beads, and prevents loss and omission of magnetic beads that may occur in the process of repeating dispersion and collection. Accordingly, the reproducibility of measurement results may be increased and a coefficient of variation (CV) may be reduced. In addition, the simplification of the washing process reduces the measurement time, and increases the throughput per hour, and enhances the performance and competitiveness of the equipment.

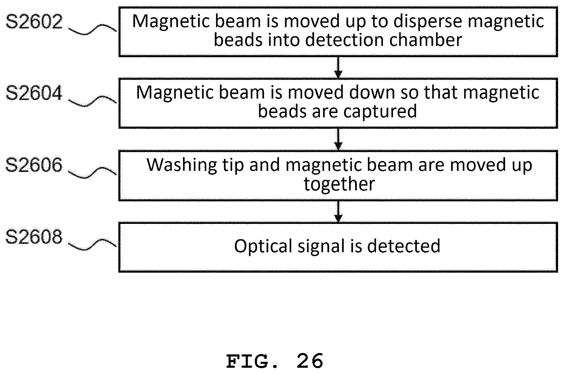

FIG. 26 is a flowchart of an optical inspection process according to an embodiment of the present invention.

In the state where the washing tip 30 is put into the detection chamber 16, the magnetic beam 564 is moved up from the washing tip 30 to disperse the magnetic bead immuno-complex into the detection chamber 16 (S2602). The magnetic bead immuno-complex is dispersed into the detection chamber (16) in a state of being bound through an immunoreaction with the detection antibody to which the enzyme is bound. The enzyme proportional to the amount of magnetic bead immuno-complex produced by binding the target material reacts with the substrate in the detection chamber 16 to generate a mineral material that can be quantitatively measured as an optical signal.

When the preset time elapses, the magnetic beam 564 is moved down to the end of the hollow part of the washing tip 30 to collect the magnetic beads on the surface of the washing tip 30 again (S2604). Next, the washing tip 30 is moved up while keeping the magnetic beam 564 at the end of the hollow part (S2606). In the state where the magnetic beads are removed in this way, an optical signal is detected by performing an optical inspection on the substrate in the detection chamber 16 (S2608).

According to this embodiment, after the reaction between the enzyme and the substrate, an optical inspection is performed in the state where the magnetic bead immuno-complex is removed. Accordingly, a bias signal or a background signal increased by the magnetic beads is reduced, and absorption and scattering of an optical signal caused by the magnetic beads are reduced. In addition, reproducibility is enhanced because the magnetic bead immuno-complex is removed and the enzyme reaction is stopped. Through this, the present embodiment can improve the immune response analysis performance.

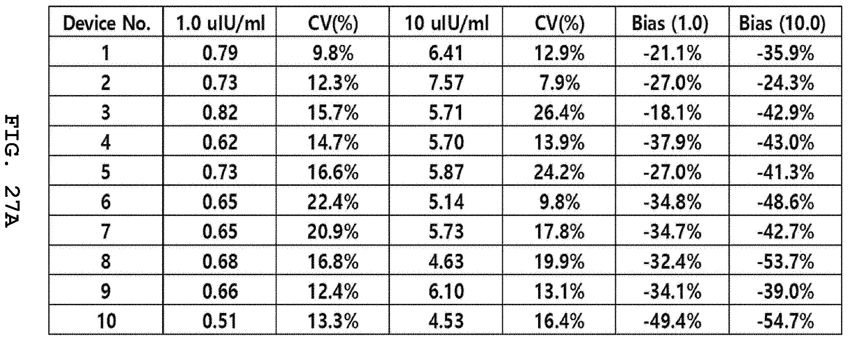

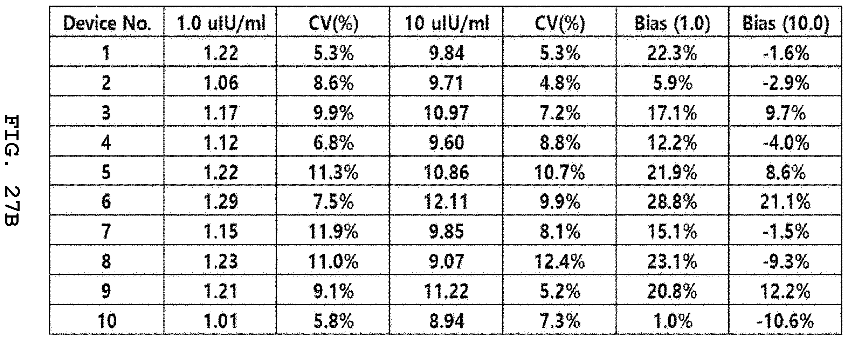

FIG. 27A shows the reproducibility and bias value of optical inspection in a state in which magnetic beads are kept in the detection chamber 16, and FIG. 27B shows the reproducibility and bias values of optical inspection in a state in which magnetic beads are removed from the detection chamber 16 according to this embodiment. Here, the bias value is calculated by ((target signal magnitude - bias signal magnitude) / (target signal magnitude)) X 100.

For example, when the concentration of the standard substance in device 10 is 1.0 µlU/ml, and magnetic beads are kept in the detection chamber 16, as shown in FIG. 27A, the coefficient of variation CV is 14.4% and the bias value is - 43.9%. However, in the state in which the magnetic beads are removed, the coefficient of variation CV decreases to 4.2% so that the reproducibility is increased, as shown in FIG 27B. Also, since the bias value is 5.7%, the Signal to Noise Ratio (SNR) increases.

Although preferred embodiments of the present invention has been described in detail, it is to be understood that the scope of the present invention is not limited thereto, and various modifications and reformations by those skilled in the art using the basic concept of the present invention as defined in the accompanying claims fall within the scope of the present invention.

## Claims

1. A method for analyzing immune response using magnetic beads, including steps of:
(a) dispersing magnetic beads in a detection chamber; and
(b) collecting the magnetic beads and separating them from the detection chamber, and then performing an optical inspection on the detection chamber.

2. The method for analyzing immune response of claim 1, further including a step of:
(c) attaching a reagent to the magnetic beads and injecting a biological sample into a reaction chamber in which the magnetic beads are stored to bind an analyte in the biological sample to the reagent in an antigen-antibody reaction.

3. The method for analyzing immune response of claim 2, further including a step of:
(d) introducing a washing tip having a magnetic beam therein into the reaction chamber to capture the magnetic beads in the reaction chamber on a surface of the washing tip.

4. The method for analyzing immune response of claim 3, further including a step of:
(e) washing a non-specific biological sample attached to the surface of the washing tip with a washing solution in a washing chamber by repeatedly moving up and down the washing tip in the washing chamber in a state where the magnetic beam is located in the washing tip.

5. The method for analyzing immune response of claim 4, wherein the step (a) of dispersing the magnetic beads includes substeps of:
(a-1) moving the washing tip from which the non-specific biological sample is washed away to the detection chamber together with the magnetic beam; and
(a-2) separating the magnetic beam from the washing tip.

6. The method for analyzing immune response of claim 5, wherein the step (b) of separating the magnetic beads from the detection chamber includes substeps of:
(b-1) attaching the magnetic beads dispersed in the detection chamber to the surface of the washing tip by placing the magnetic beam inside the washing tip; and
(b-2) moving the washing tip to which the magnetic beads are attached from the detection chamber together with the magnetic beam.

7. A method for analyzing immune response using magnetic beads, including steps of:
(a) injecting a biological sample into a reaction chamber in which magnetic beads to which reagents are attached are stored, and binding the magnetic beads with an analyte in the biological sample through an antigen-antibody reaction;
(b) moving a washing tip to the reaction chamber;
(c) positioning a magnetic beam at a bottom of the washing tip by moving the magnetic beam down into a hollow portion of the washing tip;
(d) collecting the magnetic beads in the reaction chamber by attaching the magnetic beads to the surface of the washing tip;
(e) moving the washing tip to which the magnetic beads are attached to a washing chamber;
(f) physically washing a non-specific biological sample attached to the surface of the washing tip in the washing chamber;
(g) moving the washing tip from which the non-specific biological sample is washed away to a detection chamber;
(h) dispersing the magnetic beads attached to the surface of the washing tip into the detection chamber by moving up the magnetic beam located in the hollow portion of the washing tip;
(i) attaching the magnetic beads dispersed in the detection chamber to the surface of the washing tip by moving the magnetic beam down into the hollow portion of the washing tip; and
(j) moving the washing tip to which the magnetic beads are attached from the detection chamber together with the magnetic beam, and then performing an optical inspection on the detection chamber.
